# Europäisches Patentamt
# European Patent Office
# Office européen des brevets

(19)

(11) Publication number: **0 253 579**
**B1**

(12) **EUROPEAN PATENT SPECIFICATION**

(45) Date of publication of the patent specification:
14.03.90

(51) Int. Cl.⁴: **G01N 33/53**, G01N 33/543

(21) Application number: 87306088.3

(22) Date of filing: 09.07.87

(54) Improved apparatus and process for immunoassays.

(30) Priority: 14.07.86 US 885129

(43) Date of publication of application:
20.01.88 Bulletin 88/3

(45) Publication of the grant of the patent:
14.03.90 Bulletin 90/11

(84) Designated Contracting States:
AT BE CH DE ES FR GB GR IT LI LU NL SE

(56) References cited:
EP-A- 0 186 100
WO-A-85/05451
US-A- 4 251 159

(73) Proprietor: HYBRITECH INCORPORATED,
11095 Torreyana Road, San Diego California 92126(US)

(72) Inventor: Petro-Roy, Virginia, 1221 El Mlo Drive, El Cajon California 92020(US)
Inventor: Bickenstaff, Kim D., 4819 Casals Place, San Diego California(US)

(74) Representative: Tapping, Kenneth George et al, Erl Wood Manor, Windlesham Surrey, GU20 6PH(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to an improved apparatus and process used for conducting immunoassays.

For about two decades immunoassay procedures have provided sensitive diagnostic tools for the in vivo detection of a variety of antigens associated with disease or other physical conditions of clinical significance. Originally such heterogeneous assays used a polyclonal antibody preparation bound to a solid phase. In these assays, a solution of labeled antigen is allowed to compete directly, with antigen in the sample being analyzed, for the solid phase antibody or is added to the antibody in a sequential process. The extent to which the labeled antigen is bound to the solid phase or is detected in the liquid phase can be used as a measure of the presence and quantity of antigen in the sample being analyzed.

Subsequently, non-competitive immunometric assays became available. In these assays, a polyclonal antibody preparation bound to a solid phase was also used. The sample containing the suspected antigen was allowed to contact the solid phase in order for the antigen to bind to the antibodies on the solid phase. Typically, after an incubation step the sample was separated from the solid phase which was then washed and incubated with a solution of additional polyclonal antibodies which had been labeled, for example, with a radionuclide, an enzyme, or a fluorescent moiety.

After this second incubation, the unbound labeled antibody was separated from the solid phase and the amount of labeled antibody in either the liquid phase or bound to the solid phase in an antibody:antigen:antibody sandwich was determined as a measure of the presence and/or concentration of antigen in the sample tested.

More recently, immunoassay procedures have been modified to use monoclonal antibodies. For example, U.S. Patent No. 4,376,110 describes two-site immunometric assays using pairs of monoclonal antibodies, one bound to a solid phase and the other labeled to permit detection The use of monoclonal antibody pairs which recognize different epitopic sites on an antigen has made it possible to conduct simultaneous immunometric assays in which the antigen and labeled antibody incubations do not require the intermediate washing steps of prior processes.

In the foregoing processes, the solid phase antibody is typically bound to a bead or small particles or coated on a solid surface. Alternatively, microspheres to which are bound antibody may be entrapped within a porous matrix. Recent improvements have drastically reduced the time necessary for the performance of the assays. As a result, simpler and more rapid procedures for conducting immunoassays which employ a relatively simple apparatus make such assays available for use in the physician's office and even for over-the-counter sale to lay persons for use in home health care programs. For example, U.S. Patent No. 3,811,840, Bauer et al., describes a test device for detecting low concentrations of substances in test fluids comprising an adsorbant wick having a substantially flat surface portion enclosed in a fluid impervious sheath having an aperture of predetermined limited area. The aperture is contiguous to and exposes a predetermined limited area of the flat surface portion of the wick area. Incorporated within this aperture portion of the wick is a reagent which specifically reacts with the substance being detected. In use, the device is dipped into the test fluid where the test fluid contacts the reagent area of the aperture and migrates into the remainder of the wick. Also, U.S. Patent No. 4,366,241, Tom et al., discloses an apparatus for performing immunoassays employing a device consisting of a relatively small test zone referred to as an immunoabsorbing zone, and a relatively large liquid adsorbing zone in liquid receiving relationship with the immunoabsorbing zone. At least a portion of the liquid adsorbing member about the immunoabsorbing zone is enclosed in an impermeable enclosure.

Commercially available from Hybritech Incorporated is a product sold under the trademark ICON™. This product is described in detail in U.S. Patent No. 4,632,901, Valkirs et al., issued December 30, 1986. The product employs a device which comprises a membrane, having antibody fixed to its surface, in capillary communication with an absorbent second member. The two components are enclosed in a container open at one end to permit sample to be applied to the membrane. The absorbent member, in capillary contact with the membrane, draws the applied liquid through the membrane. Air displaced within the body of the container by the addition of liquid passes through small vent ports located, for example, near the bottom or on the side of the container.

An important consideration in storing and shipping an apparatus for conducting immunoassays is that the moisture sensitive components of the apparatus be protected from contact with contaminating moisture prior to use. A method presently used is to place, for example, the Hybritech Incorporate ICON™ product, referred to above, within a hermetically sealed pouch containing desiccants well known in the art. This particular method, although well suited for its particular purposes, is expensive, adds complexity and cost to the packaging of the apparatus, and increases the size of the packaged product. Accordingly, an apparatus for conducting immunoassays which can be sealed from contaminating moisture is desirable. The product invention is directed to such an apparatus.

In general, there is provided an improved apparatus for conducting ligand-receptor assay processes wherein the apparatus comprises a test zone to which is bound a receptor; a liquid absorbing zone in liquid receiving relationship with the test zone; a container that is impervious to liquids which encloses the test zone and liquid absorbing zones; and a sealable opening in the apparatus to permit addition of assay reagents to the test zone. The apparatus is characterised in that it comprises at least one vent port in communication with the sealable opening wherein the vent port provides a means for discharging from the container gas displaced from the liquid absorbing zone. The displaced air flows in a direction opposite to the flow of assay reagents.

In a more preferred embodiment, the present invention is directed generally to the apparatus and method for conducting immunoassay processes of U.S. Patent No. 4,632,901, Valkirs et al., issued December 30, 1986. To this end, an apparatus comprising an improved container is provided for conducting immunometric assays that does not require the use of a sealed pouch to protect the moisture-sensitive membrane of the apparatus during storage prior to use. The improved container is provided with at least one vent port in communication with the liquid absorbing member and the sealable opening of the container. Air displaced within the liquid absorbing member by the addition of assay reagents passes through said vent port located within the walls of the container to the top of the container. In a more preferred embodiment, the improved container is provided with at least one vent port located in the outer surface of a removable plug, the vent port is in communication with the liquid absorbing zone and the sealable opening in the container and an additional moisture absorbing member below the liquid absorbing zone, which is for example a desiccant. The desiccant minimizes moisture that might develop in the container during manufacture or storage. As fluid reagents are added to the container during an assay procedure, air displaced within the liquid absorbing zone by the assay reagents passes through the liquid absorbing zone to the vent port and out of the sealable opening in a direction opposite the flow of the reagents. For example, the vent ports can be channels between the container and the removable plug, wherein the removable plug has vertical grooves on its outer surface that extend from the lower to the upper edges of the plug. In this way the moisture-sensitive membrane may be protected by placing a removable, for example, peelable, seal across the top of the container resulting in a smaller package form and decreased cost.

Figure 1 is an exploded perspective view of an improved apparatus for performing an immunoassay, in accordance with the present invention.

Figure 2 is a cross section taken along line 2-2 of Figure 1.

Figure 3 is an exploded perspective view of a preferred embodiment of an improved apparatus for performing an immunoassay in accordance with the present invention.

The prior art apparatus allowed the displaced gas to flow in the same direction as the flow of the assay reagents which requires a discharge opening at some distance from the opening for addition of the assay reagents.

In general, the present invention provides an improvement in an apparatus for ligand receptor assays, said apparatus comprising a test zone to which is bound a receptor, a liquid absorbing zone in liquid receiving relationship with the test zone and a container that is impervious to liquids which encloses the test zone and liquid absorbing zone, wherein the improvement comprises an improved container provided with a sealable opening in the container to permit addition of assay reagents to the test zone and at least one vent port in communication with the sealable opening wherein the vent port provides a means for discharging from the container gas displaced from the liquid absorbing zone, the displaced gas flowing in a direction opposite the flow of the assay reagents upon addition. By providing a means for discharging the displaced air from the liquid absorbing zone in such a way that it channels the air flow in a direction opposite the flow of the assay reagents, the place of discharge, i.e. the vent ports, can be located in close proximity to the opening in the container that permits addition of assay reagents to the test zone. The closer in proximity these two openings, i.e., vent port and container opening, are in the assay apparatus the easier, more convenient, and less expensive it is to seal both openings simultaneously and protect the moisture sensitive components of the apparatus.

The present invention is an improvement in an apparatus for liquid receptor assay, said apparatus comprising as a first member, a porous membrane or filter to which is bound receptor or anti-receptor for a ligand, or which is capable of filtering cellular material from a sample being assayed if the ligand is associated with the cellular material, a second member which is an absorbent member having capillary passageways generally transverse to the upper and lower surfaces of said second member, and a container (the apparatus is set forth in greater detail in U.S. Patent No. 4,632,901, Valkirs et al ., issued December 30, 1986) wherein the improvement comprises an improved apparatus with at least one vent port in communication with the liquid absorbing second member and the top of the container. Air displaced within the liquid absorbing zone by the addition of assay reagents passes out of the liquid absorbing zone and through said vent port located between the wall of the container and the liquid absorbing zone to the top of the container. Accordingly, the entire container, including the vent port, can be sealed with a single removable seal. Thus, the moisture-sensitive membrane may be protected by placing a removable seal across the top of the container resulting in a smaller package size and decreased cost.

As noted above, the apparatus of the present invention comprises, as a first member, a porous membrane or filter to which is bound receptor or anti-receptor for a ligand, or which is capable of filtering cellular material from a sample being assayed where the ligand is associated with the cellular material. In the latter case, the membrane or filter is selected to have a pore size which permits this separation. Any of a variety of filtering members may be used including glass fiber filters and filters of various synthetic or natural materials.

When the porous member has receptor bound to it, the receptor is selected for its ability to selectively bind directly with the target ligand (i.e., analyte). For example, if the ligand is an antigen, the receptor may be an antibody, preferably a monoclonal antibody. If the target ligand is an antibody, the receptor may be an antigen or anti-antibody. If the lig-

and is an enzyme, the receptor may be a receptor for the enzyme. If the ligand is a nucleic acid, for example, RNA or DNA, the receptor may be a complementary oligomer of DNA or RNA. In a preferred embodiment, the first member is a membrane or filter to which an antibody preparation is covalently bound. Preferably, the antibody preparation comprises a monoclonal antibody even though polyclonal antibodies from anti-sera may be used. Techniques for polyclonal and monoclonal antibody preparation are now well known in the art. For example, preparatrion of polyclonal antibodies is disclosed in Freund, J., and McDermott, K., Proc. Soc. Exp. Biol. Med, 49:548 (1942) (describes use of Freund's "complete" adjuvant) and Freund, J. and Walter, A.W., Proc. Soc. Exp. Biol. Med., 56:47 (1944) (describes use of Freund's "incomplete" adjuvant). The preparation of monoclonal antibodies now is well-known to those skilled in the art and is described in detail in Kohler, G., and Milstein, C., Nature, 256:495 (1975).

The material of the porous member is selected from a material to which the receptor or, anti-receptor can be bound. In the case of protein receptors or anti-receptors, e.g., anti-antibodies or antigens, a preferred material is nylon which contains amino group residues or into which such groups have been introduced by chemical means, which permit a protein to be coupled to it by the well known glutaraldehyde method. Antibodies can be coupled to glass fibers through aminosilanes. Other natural or synthetic materials which can be coupled directly or through intermediates to a receptor may also be used.

The foregoing stresses chemical binding of the receptor or anti-receptor to the porous member. However, in appropriate cases the receptor or anti-receptor may be coated on the porous member or be a particulate, e.g., microspheres, which is entrapped within the interstices of the porous member. Therefore, as used, the term "bound" is intended to embrace any means for fixing receptor or anti-receptor to the porous member.

The second member is an absorbent member having capillary passageways generally transverse to the upper and lower surfaces. The second member is assembled with the first in a manner which permits capillary communication between the pores or interstices of the first member and the capillaries of the second member. Thus, as a liquid is applied to the first member and saturates it, the liquid is drawn into the second member. As a result, flow can be induced through the first member when a liquid sample is applied to the upper surface of the first member even though the hydrostatic pressure of the fluid is so low that unaided it could not flow through the first member without the application of pressure to force it through or a vacuum to draw it through.

The selection of material for the second member is not critical and a variety of fibrous filter materials can be used. A useful material is cellulose acetate fibers oriented as in a cigarette filter. Those skilled in the art will appreciate that other absorbent members made of polyester, polyolefin or other materials may be used in place of cellulose acetate.

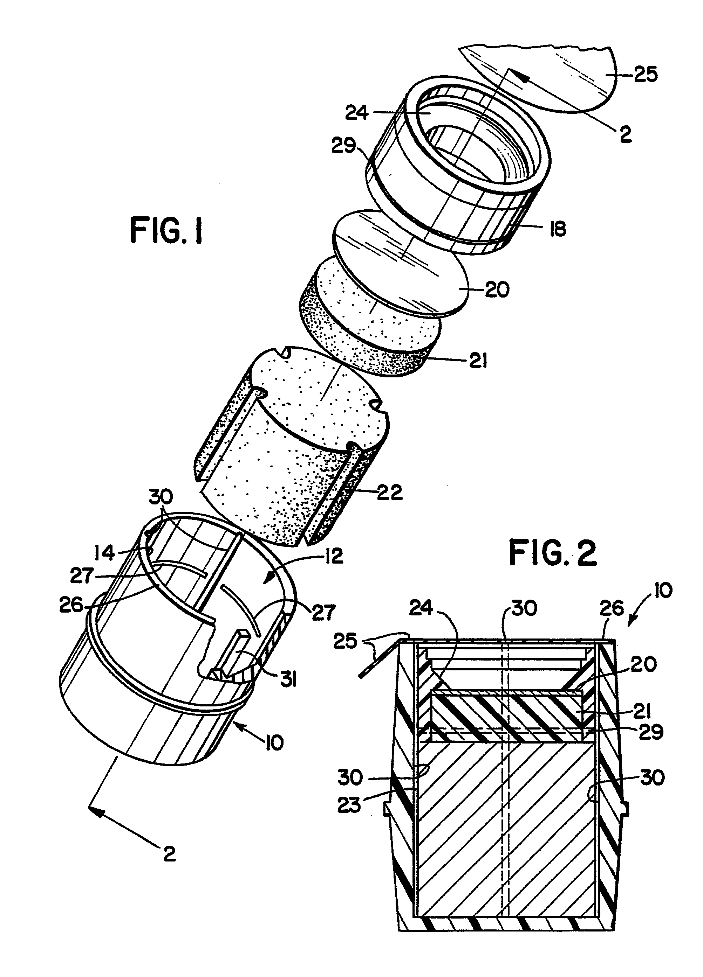

Figure 1 shows an exploded perspective view of the apparatus of the present invention. Thus, in Figure 1, a cylindrical container 10, although it may have any other appropriate shape, is closed at the bottom and is provided with an upper opening 12 defined by sidewall 14. The container is impervious to liquids and may be made, for example, of glass or a suitable plastic material. As shown in Figure 1, removable plug 18 is insertable through opening 12 in container 10 to permit insertion of the porous member 20, a circular membrane or filter disc, and an optional member 21, which rests on cylindrical absorbent member 22, which is also inserted through opening 12.

A portion of removable plug 18 is constricted as shown in Figure 1 by reference number 24 to provide an integral funnel to direct sample onto the member 20 and to assure that effective washing of sample and other reagents added to the member 20 is accomplished.

The size of member 22, and, therefore, the volume of the portion of container 10 below the constriction is preferably selected so that all of the liquid to be added to the apparatus during an assay can be received in and retained in absorbent member 22. Means for venting air consisting of at least one vent port 23 (illustrated in Figure 2) is provided. Vent ports 23 are formed of grooves 30 in container 10 which leave a space between the inner surface of container 10 and the outer surface of removable plug 18, to allow air displaced by the addition of liquid to the apparatus to be channeled out through opening 12 in container 10. In this way, the entire container 10 can be sealed from the environment with removable seal 25, removably attached over opening 12 of container 10 at surface 26. This seal may be made under such conditions of ambient moisture as are required. Requirements relating to moisture content of the sealed apparatus are well known in the art and need no further explanation here. Upon application of removable seal 25 as detailed above to container 10 having a closed bottom end as depicted in Figure 2, a hermetic moisture resistant seal is obtained which may protect the contents of container 10 from contamination by exposure to moisture during the period of time from manufacture to use. As is well known in the art, removable seals such as removable seal 25 have the characteristic of being less expensive and less space consuming than pouch seals.

Protrusions 27 of container 10 engage annular groove 29 of removable plug 18 to lock removable plug 18 in place within container 10.

Supports 31 provide support to optional member 21, porous member 20 and removable plug 18 to prevent items 18, 20 and 21 from entering container 10 more than a desired amount.

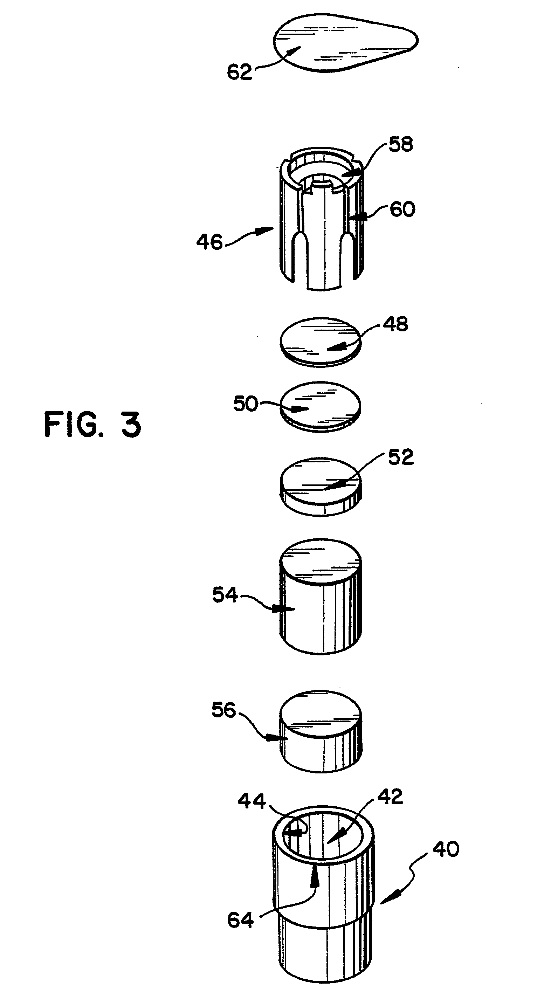

Figure 3 is an exploded perspective of a preferred apparatus of this invention. Thus, in Figure 3, a cylindrical container 40, although it may have any other appropriate shape, is closed at the bottom and is provided with an upper opening 42 defined by sidewall 44. The container is impervious to liquids and may be made, for example, of glass or a suitable plastic material. A removable plug 46 is insertable through opening 42 in container 40 and encompasses the porous member 48, a circular membrane or filter disc, optional porous members 50 and 52, whose function is described below, cylindrical absorbent member 54 and optional moisture absorbent member 56 within container 40.

A portion of removable plug 46 is constructed to provide an integral funnel to direct sample onto the member 48, and to assure the effective washing of sample and other reagents added to the member 48.

The size of member 54, and, therefore, the volume of the portion of container 40 below the constriction 58 is preferably selected so that all of the liquid to be added to the apparatus during an assay can be received in and retained in absorbent member 54. Means for venting air consisting of at least one vent port 60 (illustrated in Figure 3) is provided. Vent port 60 is formed of grooves in removable plug 46 which leave a space between the inner surface of container 40 and the outer surface of removable plug 46, to allow air displaced from member 54 by the addition of liquid to the apparatus to be channeled out through opening 42 in container 40. In this way, the entire container 40 can be sealed from the environment with removable seal 62, removably attached over opening 42 of container 40 at surface 64. This seal may be made under such conditions of ambient moisture as are required. Requirements relating to moisture content of the sealed apparatus are well known in the art and need no further explanation. Upon application of removable seal 62, as detailed above, to container 40 having a closed bottom end as depicted in Figure 3, a hermetic moisture resistant seal is obtained which may protect the contents of container 40 from contamination by exposure to moisture during the period of time from manufacture to use. As is well known in the art, removable seals such as removable seal 62 have the characteristic of being less expensive and less space consuming than pouch seals.

We have found that when cellulose acetate is used as a material for the second absorbent member 54 it may bind labeled receptor non-specifically at its upper surface. Accordingly, this non-specific binding may cause some visual color change to occur at the upper surface of the second absorbent member just under the first member 48. To avoid this color change being visualized through the first member 48, a third porous member (designated 52 in Figure 3) of porous polyethylene or other material which does not bind receptor non-specifically is preferably placed between members 48 and 54. Although the non-specific binding may still occur on the upper surface of the second absorbent member 54, the third porous member 52 functions as an optical block so that whatever non-specific binding occurs cannot be visualized through the first member 48. Additionally, the third porous member 52 can function as a support means for the first porous member 48.

We have found also that incomplete washing results in a modest color development problem which is exacerbated when the device is subject to high or low temperatures during shipping or storage. The cause of this problem is believed to be the buckling or lifting of the first porous member 48 from the upper surface of the third porous member 52, creating air gaps between the first and third members and reducing the uniform capillary communication between the first porous member and the second absorbent member 54. As a consequence, labeled receptor is not washed from various areas of the first member 48 and blotchy color development results. Although the applicants do not wish to be bound by any particular theory, applicants believe that the buckling or lifting of the first member 48 is caused by different co-efficients of expansion of the first member 48 and the removable plug 46. In other words, because the first member 48 is held in place by the edges of the removable plug 46 and these two elements of the device expand and contract unequally in response to changes in temperature, the shifting position of the two elements may cause the first member to buckle, creating air gaps between the first member and the third member. To minimize this problem we have found that an optional fourth porous member (designated 50 in Figure 3) of polyester/cellulose, polyester, cotton, or other materials which are compressible, resilient and hydrophilic is preferably placed between members 48 and 52. The fourth porous member expands and contracts to fill any gaps that may develop between members 48 and 52. This fourth porous member may be selected from any material that is porous, does not bind receptor non-specifically and is expandable and conforming enough to fill the gaps between the first and third members thereby maintaining capillary communication between the first porous member, the third porous member, the second absorbent member. Hydrophilic properties of the material selected for the fourth member may be enhanced by coating the material with a surfactant.

As previously mentioned, the components of the described ligand receptor assay apparatus are sensitive to contamination by moisture. Accordingly, an optional fifth member 56 can be inserted within container 40 below the second absorbent member 54 and is selected to be made material that can absorb any moisture that may exist within the apparatus. For example, member 56 may be selected from desiccants well known to one of skill in the art.

Thus, an improved apparatus for use in performing immunoassays is disclosed which utilizes an improved design to allow the entire apparatus to be sealed by a single removable seal over the top.

## Claims

1. An apparatus for use in a ligand-receptor assay process for the detection of a target analyte in a fluid sample, said apparatus comprising:

a) a test zone (20) to which is bound a receptor or anti-receptor;

b) a liquid absorbing zone (22) in liquid receiving relationship with said test zone;

c) a container (10) that is impervious to liquids which encloses said test zone and said liquid absorbing zone; and

d) a sealable opening (12) in said container to permit addition of assay reagents to said test zone;

characterized in that it comprises at least one vent port (23) in communication with said sealable opening (12) wherein said vent port provides a means for discharging from said container gas displaced from said liquid absorbing zone, the flow of said gas being in a direction opposite to the flow of said assay reagents through said liquid absorbing zone.

2. An apparatus as claimed in Claim 1, which comprises a removable seal (25) sealing said sealable opening.

3. An apparatus as claimed in Claim 1 or 2 in which said receptor is an antibody, anti-antibody, or an antigen.

4. An apparatus as claimed in Claim 3 in which the antibody is a monoclonal antibody.

5. An apparatus according to Claim 1, wherein said test zone is separated from said liquid absorbing zone by a porous member (21).

6. An apparatus as claimed in any one of Claims 1 to 5, wherein said sealable opening further comprises a section having sides (24) which slope inwardly to define a funnel for direction of the added reagents onto the upper surface of the test zone (20).

7. An apparatus as claimed in Claim 6, wherein said test zone member (20) is a porous matrix in which are entrapped microspheres to which are bound receptors against said target analyte.

8. An apparatus as claimed in Claim 1 comprising:
a) a first member (20) which is porous and to which is bound a receptor or anti-receptor, or which is capable of separating cellular material with which the ligand is associated from the fluid sample, which member has upper and lower surfaces;
b) a second member (22) which is a body of absorbent material having a surface over which the first member is placed and having capillaries therethrough in a direction generally transverse to the surface over which the first member is placed which capillaires are in communication with the pores on the lower surface of the first member so as to draw fluid added to the upper surface which has permeated the first member into the capillaries of the second member;
c) a container (10) that is impervious to liquid which encloses said first and second members, and
d) a sealable opening (12) in said container to permit addition of assay reagents to said upper surface of said first member;
wherein there is provided a removable seal (25) sealing said sealable opening; and at least one vent port (23) in communication with said second member and said sealable opening.

9. An apparatus as claimed in Claim 1 comprising:
a) a first member (48) which is porous and to which is bound a receptor or anti-receptor, or which is capable of separating cellular material with which the target analyte is associated from the fluid sample, which member has upper and lower surfaces;
b) a second member (54) which is a body of absorbent material having a surface over which the first member is placed and having capillaries therethrough in a direction generally transverse to the surface over which the first member is placed, said capillaries are in communication with the pores of the lower surface of said first member so as to draw fluid added to the upper surface which has permeated said first member into the capillaries of the second member;
c) a third porous member (52) separating said first porous member from said second porous member;
d) a fourth porous member (50) separating said first porous member from said third porous member;
e) a fifth member (56) placed placed below said second member providing a means for absorbing moisture;
f) a container (40) that is impervious to liquids which encloses said first, second, third, fourth, and fifth members, and
1) a sealable opening (42) in said container to permit addition of assay reagnet so said upper surface of said first member;
2) a removable seal (62), sealing said sealable opening;
3) a removable plug (46) encompassing said first, second, third, fourth, and fifth members, said plug fitting within said container and having at least one groove (60) on its outer surface extending from the lower to upper edges of said plug, said groove providing a means for channeling air displaced from the second absorbent member to said sealable opening.

**Patentansprüche**

1. Gerät zur Verwendung in einem Ligand-Rezeptor-Untersuchungsverfahren für die Ermittlung eines Zielanalytikums in einer flüssigen Probe, enthaltend:
a) eine Testzone (20), an die ein Rezeptor oder Antirezeptor gebunden ist;
b) eine Flüssigkeitsabsorptionszone (22), die in flüssigkeitsaufnehmenden Verhältnis zu der genannten Testzone steht;
c) einen Behälter (10), der für Flüssigkeiten undurchlässig ist und der die Testzone und die Flüssigkeitsabsorptionszone umschließt; und
d) eine verschließbare Öffnung (12) in dem Behälter, um die Hinzufügung von Untersuchungsreagenzien zu der Testzone zu ermöglichen;
dadurch gekennzeichnet, daß es enthält: wenigstens eine Entlüftungsöffnung (23), die mit der verschließbaren Öffnung (12) in Verbindung ist, wobei die genannte Entlüftungsöffnung eine Einrichtung zum Abgeben von Gas aus dem Behälter darstellt, das aus der Flüssigkeitsabsorptionszone verdrängt wird, wobei die Strömung dieses Gases in einer Richtung verläuft, die entgegengesetzt zu der Strömung der Untersuchungsreagenzien durch die Flüssigkeitsabsorptionszone ist.

2. Gerät nach Anspruch 1, das einen abnehmbaren Verschluß (25) enthält, der die verschließbare Öffnung verschließt.

3. Gerät nach Anspruch 1 oder 2, bei dem der Rezeptor ein Antikörper, Anti-Antikörper oder ein Antigen ist.

4. Gerät nach Anspruch 3, bei dem der Antikörper ein monokloner Antikörper ist.

5. Gerät nach Anspruch 1, bei dem die Testzone von der Flüssigkeitsabsorptionszone durch ein poröses Element (21) getrennt ist.

6. Gerät nach einem der Ansprüche 1 bis 5, bei dem die verschließbare Öffnung weiterhin einen Abschnitt enthält, der Seiten (24) aufweist, die nach innen zusammenlaufen, um einen Trichter zu bilden, um die hinzugefügten Reagenzien auf die Oberseite der Testzone (20) zu richten.

7. Gerät nach Anspruch 6, bei dem das Testzonenelement (20) eine poröse Matrix ist, in der Mikrokügelchen eingeschlossen sind, an die Rezeptoren gegen das Zielanalytikum gebunden sind.

8. Gerät nach Anspruch 1, enthaltend:

a) ein erstes Element (20), das porös ist und an das ein Rezeptor oder Antirezeptor gebunden ist oder das in der Lage ist, zellulares Material, dem der Ligand zugeordnet ist, aus der flüssigen Probe abzutrennen, welches Element Ober- und Unterseiten aufweist;

b) ein zweites Element (22), das ein Körper aus absorbierendem Material ist, das eine Fläche aufweist, über der das erste Element angeordnet ist, und das durchgehende Kapillaren in einer Richtung hat, die im wesentlichen quer zu der Oberfläche ist, über der das erste Element angeordnet ist, wobei die Kapillaren mit den Poren an der Unterseite des ersten Elements in Verbindung stehen, um der Oberseite zugeführte Flüssigkeit, die das erste Element durchdrungen hat, in die Kapillaren des zweiten Elementes anzuziehen;

c) einen Behälter (10), der für Flüssigkeit undurchlässig ist und der die ersten und zweiten Elemente umschließt, und

d) eine verschließbare Öffnung (12) in dem Behälter, um die Hinzufügung von Untersuchungsreagenzien zu der Oberseite des ersten Elements zu ermöglichen, wobei vorgesehen ist:

ein abnehmbarer Verschluß (25), der die verschließbare Öffnung verschließt; und wenigstens eine Entlüftungsöffnung (23), die mit dem zweiten Element und mit der verschließbaren Öffnung verbunden ist.

9. Gerät nach Anspruch 1, enthaltend:

a) ein erstes Element (48), das porös ist und an das ein Rezeptor oder ein Antirezeptor gebunden ist oder das in der Lage ist, zellulares Material, dem das Zielanalytikum zugeordnet ist, aus der flüssigen Probe abzutrennen, welches Element Ober- und Unterseiten hat;

b) ein zweites Element (54), das ein Körper aus absorbierendem Material ist und eine Oberfläche hat, über der das erste Element angeordnet ist, und das durchgehende Kapillaren hat, die in einer Richtung verlaufen, die im wesentlichen quer zu der Oberfläche ist, über der das erste Element angeordnet ist, wobei die Kapillaren in Verbindung mit den Poren der Unterseite des ersten Elements sind, um Flüssigkeit, die der Oberseite zugeführt worden ist und das erste Element durchdrungen hat, in die Kapillaren des zweiten Elements zu ziehen;

c) ein drittes poröses Element (52), das das erste poröse Element von dem zweiten porösen Element trennt;

d) ein viertes poröses Element (50), das das erste poröse Element von dem dritten porösen Element trennt;

e) ein fünftes Element (56), das sich unter dem zweiten Element befindet und eine Einrichtung zum Absorbieren von Feuchtigkeit darstellt;

f) einen Behälter (40), der für Flüssigkeiten undurchlässig ist, und der die ersten, zweiten, dritten, vierten und fünften Elemente umschließt, und

1) eine verschließbare Öffnung (42) in dem Behälter um eine Hinzufügung von Untersuchungsreagenzien zur Oberseite des ersten Elements zu ermöglichen;

2) einen abnehmbaren Verschluß (62), der die verschließbare Öffnung verschließt;

3) einen abnehmbaren Stopfen (46), der die ersten, zweiten, dritten, vierten und fünften Elemente umgibt, welcher Stopfen in den Behälter paßt und wenigstens eine Rille (60) an seiner Außenseite aufweist, die sich von dem unteren zum oberen Rand des Stopfens erstreckt, welche Rille eine Einrichtung zum Durchleiten von Luft darstellt, die aus dem zweiten absorbierenden Element zur verschließbaren Öffnung verdrängt wird.

**Revendications**

1. Appareil destiné à un procédé de dosage de récepteurs de ligands pour la détection d'un analyte-cible au sein d'un échantillon liquide, cet appareil comprenant:

a) une zone d'essai (20) à laquelle est lié un récepteur ou un anti-récepteur;

b) une zone d'absorption de liquide (22) se trouvant en relation de réception de liquide avec cette zone d'essai;

c) un récipient (10) imperméable aux liquides, qui renferme cette zone d'essai et cette zone d'absorption de liquides; et

d) une ouverture (12) pouvant être étanchée pratiquée dans ce récipient en vue de pouvoir ajouter des réactifs de dosage à cette zone d'essai;

caractérisé en ce qu'il comprend au moins un orifice d'aération (23) se trouvant en communication avec cette ouverture (12) pouvant être étanchée, cet orifice d'aération fournissant un moyen en vue d'évacuer hors de ce récipient des gaz déplacés depuis la zone d'absorption de liquides, le flux de ces gaz s'écoulant en direction opposée au flux des réactifs de dosage à travers la zone d'absorption de liquides.

2. Appareil selon la revendication 1, muni d'un joint étanche amovible (25) destiné à l'ouverture pouvant être étanchée.

3. Appareil selon la revendication 1 ou 2, dans lequel le récepteur est un anticorps, un anti-anticorps ou un antigène.

4. Appareil selon la revendication 3, dans lequel l'anticorps est un anticorps monoclonal.

5. Appareil selon la revendication 1, dans lequel la zone d'essai est séparée de la zone d'absorption de liquides via un élément poreux (21).

6. Appareil selon l'une quelconque des revendications 1 à 5, dans lequel l'ouverture pouvant être étanchée est munie, en outre, d'une section présentant des côtés (24) inclinés vers l'intérieur, en vue de définir un entonnoir destiné à diriger les réactifs ajoutés, jusque sur la surface supérieure de la zone d'essai (20).

7. Appareil selon la revendication 6, dans lequel l'élément (20) de la zone d'essai est une matrice poreuse dans laquelle sont emprisonnées des microsphères auxquelles sont liées des récepteurs à l'encontre de cet analyte-cible.

8. Appareil selon la revendication 1, cet appreil comprenant:

a) un premier élément (20) poreux et auquel est lié un récepteur ou un anti-récepteur, ou bien qui est apte à séparer de la matière cellulaire à laquelle est associé le ligand se trouvant au sein de l'échantillon liquide, cet élément étant muni de surfaces supérieure et inférieure;

b) un second élément (22), à savoir un corps constitué d'une matière absorbante et muni d'une surface par-dessus laquelle est disposé le premier élément, ce corps comprenant des capillaires le traversant dans un sens généralement transversal, et s'étendant en direction de la surface par-dessus laquelle on a disposé le premier élément, les capillaires se trouvant en communication avec les pores situés sur la surface inférieure du premier élément, de façon à attirer le liquide ajouté à la surface supérieure et qui a imprégné le premier élément, jusque dans les capillaires du second élément;

c) un récipient (10) imperméable aux liquides, et que renferme les premier et second éléments, et

d) une ouverture (12) pouvant être étanchée, pratiquée dans ce récipient, en vue de permettre l'addition des réactifs de dosage à la surface supérieure de ce premier élément; tandis que cet appareil est muni d'un joint étanche amovible (25) destiné à cette ouverture pouvant être étanchée; et d'au moins un orifice d'aération (23) se trouvant en communication avec le second élément et l'ouverture pouvant être étanchée.

9. Appareil selon la revendication 1, comprenant:

a) un premier élément (48) poreux et auquel est lié un récepteur ou un anti-récepteur, ou bien qui est apte à séparer de la matière cellulaire à laquelle est associé le ligand se trouvant au sein de l'échantillon liquide, cet élément étant muni de surfaces supérieure et inférieure;

b) un deuxième élément (54), à savoir un corps constitué d'une matière absorbante et muni d'une surface par-dessus laquelle est disposé le premier élément, ce corps comprenant des capillaires le traversant dans un sens généralement transversal, et s'étendant en direction de la surface par-dessus laquelle on a disposé le premier élément, les capillaires se trouvant en communication avec les pores situés sur la surface inférieure du premier élément, de façon à attirer le liquide ajouté à la surface supérieure et qui a imprégné le premier élément, jusque dans les capillaires du deuxième élément;

c) un troisième élément poreux (52) séparant le premier élément poreux du deuxième élément poreux;

d) un quatrième élément poreux (50) séparant le premier élément poreux du troisième élément poreux;

e) un cinquième élément (56) disposé en dessous de ce deuxième élément en vue de fournir un moyen destiné à absorber l'humidité;

f) un récipient (40) imperméable aux liquides, renfermant les premier, deuxième, troisième, quatrième et cinquième éléments, ainsi que

1) une ouverture (42) pouvant être étanchée, pratiquée dans ce récipient en vue de permettre l'addition de réactifs de dosage à la surface supérieure de ce premier élément;

2) un joint étanche amovible (62) destiné à l'ouverture pouvant être étanchée;

3) un bouchon amovible (46) englobant les premier, deuxième, troisième, quatrième et cinquième éléments, ce bouchon venant d'adapter au sein du récipient, et étant muni d'au moins une rainure (60) sur sa surface externe, cette rainure s'étendant depuis les bords inférieurs jusqu'aux bords supérieurs de ce bouchon, tandis qu'elle fournit un moyen destiné à canaliser de l'air déplacé depuis le deuxième élément absorbant jusqu'à l'ouverture pouvant être étanchée.

FIG. 1

FIG. 2

FIG. 3